# EUROPEAN PATENT SPECIFICATION

(11) **EP 0 644 193 B1**
(45) Date of publication and mention of the grant of the patent: **26.04.2000**
(21) Application number: 94114607.8
(22) Date of filing: 16.09.1994
(51) Int. Cl.: C07D 473/40

(54) **Process for preparing 2-acetylamino-6-chloropurine**
Verfahren zur Herstellung von 2-Acetylamino-6-chloropurin
Procédé pour la préparation du 2-acétylamino-6-chloropurine

(30) Priority: 17.09.1993 JP 25496093; 25.08.1994 JP 22552694
(43) Date of publication of application: 22.03.1995
(73) Proprietor: JUZEN CHEMICAL CO.LTD., Toyama-shi, Toyama-ken (JP)
(72) Inventor: Miyata, Yasuhiro, Nei-gun, Toyama-ken (JP); Kosumi, Hideyuki, Toyama-shi, Toyama-ken (JP); Nomura, Yosihisa, Toyama-shi, Toyama-ken (JP)
(74) Representative: Casalonga, Axel

(56) References cited:
- EP-A- 0 203 685
- EP-A- 0 433 845
- EP-A- 0 433 846
- EP-A- 0 590 361
- WO-A-92/13859
- WO-A-93/15075
- SYNOWIETZ, C., ET.AL.: "Chemiker Kalender" 3. ED., , SPRINGER-VERLAG, BERLIN

## Description

This invention relates to a process for preparing 2-acetylamino-6-chloropurine which is well known as an intermediate having wide utility in the field of synthesis of nucleosides in the pharmaceutical industry.

2-Acetylamino-6-chloropurine has been hitherto prepared according to the processes set forth, for example, in Japanese Laid-open Patent Application No. 4-234883, International Laid-open Patent Application Nos. WO92/13859, WO93/15075 and European Laid-open Patent Application EP 0590361A1. In these processes, 2,9-diacetylguanine or 2-acetylguanine is reacted with a halogenating agent using, as a catalyst, a quaternary ammonium salt such as tetraethylammonium chloride, triethylmethylammonium chloride or benzyltriethylammonium chloride, or a triethylamine hydrochloride and a tertiary amine, followed by hydrolysis to obtain intended 2-acetylamino-6-chloropurine.

However, the catalysts used in the above processes are expensive and are difficult to recover. With the industrial preparation, costs for starting materials become high, resulting in inconvenient high production costs.

It is accordingly an object of the invention to provide a process for preparing 2-acetylamino-6-chloropurine which overcomes the problem involved in the prior art processes.

It is another object of the invention to provide a process for preparing 2-acetylamino-6-chloropurine which makes use of catalysts which are inexpensive or which can be readily recovered whereby production costs can be conveniently reduced, with an intended product of high quality being obtained at a yield higher than in prior art counterparts. * The above objects can be achieved, according to the invention, by a process which comprises suspending 2,9-diacetylguanine or 2-acetylguanine (N²-acetylguanine) in a polar inert solvent, reacting the 2,9-diacetylguanine or 2-acetylguanine with phosphorus oxychloride as a halogenating agent in the presence of a catalyst which consists of a combination of a water-insoluble tertiary amine and ammonium chloride or a combination of a water-insoluble tertiary amine and a hydrochloride thereof, and subjecting the resulting reaction product to hydrolysis to obtain 2-acetyiamino-6-chloropurine.

According to the invention as claimed this tertiary amine is selected from the group tripopylamine, tributylamine, N,N-dimethylaniline and N,N-diethylaniline.

Preferably, the process of the invention comprises:
suspending 2,9-diacetylguanine or 2-acetylguanine in a polar inert solvent such as, for example, acetonitrile, tetrahydrofuran or dioxane;
adding a catalyst consisting of above-indicated water-insoluble tertiary amine and ammonium chloride or a hydrochloride of the tertiary amine;
further adding phosphorus oxychloride to the resultant mixture; reacting the 2,9-diacetylguanine or 2-acetylguanine with the phosphorus oxychloride at a temperature ranging from 20°C to 80°C;
subjecting the resultant reaction solution to hydrolysis by adding an alkaline aqueous solution such as, for example, ammonia water or a caustic soda aqueous solution so that the reaction solution is adjusted in pH to 2 to 9; and at a temperature of 0°C to 70° C;
and collecting and purifying the resulting reaction product from the solution to obtain highly pure 2-acetylamino-6-chloropurine.

In the practice of the invention, the catalyst may be made of either a combination of a water-insoluble tertiary amine and ammonium chloride, or a combination of a water-insoluble tertiary amine and a hydrochloride thereof. The tertiary amine which is insoluble in water is N,N-dimethylaniline, N,N-diethylaniline, tripropylamine or tributylamine. The molar ratio of the tertiary amine to 2,9-diacetylguanine or 2-acetylguanine is preferably in the range of 1 to 5:1. When ammonium chloride is used, the molar ratio of ammonium chloride to 2,9-diacetylguanine or 2-acetylguanine is preferably in the range of 0.5 to 5:1. Where a hydrochloride of the tertiary amine is used instead of ammonium chloride, the molar ratio of the hydrochloride to 2,9-diacetylguanine or 2-acetylguanine is preferably in the range of 1 to 5:1.

Moreover, the phosphorus oxychloride which is reacted with 2,9-diacetylguanine or 2-acetylguanine should preferably be used at a molar ratio, to 2,9-diacetylguanine or 2-acetylguanine, of 2 to 10:1.

The tertiary amine used for the reaction, i.e. N,N-dimethylaniline, N,N-diethylaniline,tripropylamine or tributylamine, and a corresponding hydrochloride of the tertiary amine can be recovered in the form of a tertiary amine by neutralization and subsequent purification procedures such as distillation under reduced pressure. Where the tertiary amine and ammonium chloride are used, in combination, as a reaction catalyst, the tertiary amine alone can be collected by the procedure set out hereinabove. It will be noted here that ammonium chloride which cannot be recovered is far more inexpensive than the tertiary amine.

The hydrochlorides of the tertiary amines insoluble in water and used as one of the catalytic components, i.e. N,N-dimethylaniline hydrochloride, N,N-diethylaniline hydrochloride, tripropylamine hydrochloride or tributylamine hydrochloride, can be readily, industrially obtained by blowing a hydrochloric acid gas in the dimethylaniline, diethylaniline, tripropyl- or tributylamine, or by adding water to a mixed solution of N,N-dimethylaniline, N,N-diethylaniline tripropyl- or tributylamine and phosphorus oxychloride.

The invention is described in more detail by way of examples which should not be construed as limiting the invention thereto.

### Example 1

108.2 g of N,N-dimethylaniline and 20 ml of acetonitrile were charged and cooled under agitation, into which 32.6 g of hydrochloric acid gas was introduced while weighing, followed by further addition of 70.0 g of 2,9-diacetylguanine (product of Juzen Chem. Co., Ltd.), 54.1 g of N,N-dimethylaniline, 330 ml of acetonitrile and 136.9 g of phosphorus oxychloride for reaction at 55°C to 60°C for 3 hours under agitation. Subsequently, while keeping at 30°C or below, the resultant reaction solution was adjusted in pH to 5 to 6 by use of a 30% caustic soda aqueous solution, followed by collection of crude crystals of 2-acetylamino-6-chloropurine. The crude crystals were suspended in water and then subjected to purification with 25% aqueous ammonia and 35% hydrochloric acid at 50°C or below, followed by filtration of the crystals, washing with water and drying to obtain 50.3 g of 2-acetylamino-6-chloropurine with a purity of 99.8% (as analyzed by liquid chromatography) (yield 79.8%).

### Example 2

48.0 g of N,N-diethylaniline, 19.6 g of phosphorus oxychloride and 50 ml of acetonitrile were mixed under agitation, into which 2.3 g of water was dropped while cooling. Thereafter, 10.0 g of diacetylguanine was added to the mixture, followed by heating to 40°C. Thereafter, 19.6 g of phosphorus oxychloride was dropped in about 30 minutes, followed by reaction at 55°C to 60°C for 2 hours. The resultant reaction solution was treated for purification in the same manner as in Example 1 to obtain 6.5 g of 2-acetylamino-6-chloropurine with a purity of 99.4 % (yield 72.2%).

### Example 3

10.0 g of diacetylguanine, 1.2 g of ammonium chloride and 15.5 g of dimethylaniline were charged, to which 50 ml of acetonitrile and 19.6 g of phosphorus oxychloride were added, followed by reaction at 55°C to 60°C for 5 hours under agitation. The resultant reaction solution was treated for purification in the same manner as in Example 1 to obtain 6.2 g of 2-acetylamino-6-chloropurine with a purity of 99.2 % (yield 68.9%).

### Example 4

3.5 g of 2-acetylguanine, 9.5 g of N,N-diethylaniline hydrochloride, 3.8 g of N,N-diethylaniline and 20 ml of acetonitrile were charged, to which 6.5 g of phosphorus oxychloride was added, followed by reaction at 55°C to 60°C for 5 hours under agitation. The resultant reaction solution was treated for purification in the same manner as in Example 1 to obtain 2.9 g of 2-acetylamino-6-chloropurine with a purity of 99.0% (yield 75.6%).

### Example 5

### Hydrolysis of 2-acetylamino-6-chloro-purine.

20 g of the 2-acetylamino-6-chloropurine obtained in Example 1 was added to a 5% caustic soda aqueous solution and agitated at 20°C to 50°C for 1 to 5 hours. Diluted sulfuric acid was added in order to adjust the pH of the solution to 5 to 7, after which agitation was continued for 1 hour, followed by filtration of the resultant crystals, washing them with water and drying to obtain 15.6 g of 2-amino-6-chloropurine with a purity of 99.1% (analyzed by liquid chromatography) (yield 77.7%). Thus, 2-amino-6-chloropurine which is known as an intermediate for guanine nucleoside analogues important as medicines could be obtained by subjecting 2-acetylamino-6-chloropurine to an ordinarily employed hydrolysis procedure.

As will be apparent from the foregoing, according to the process of the invention, general-purpose, inexpensive ammonium chloride and a water-insoluble tertiary amine, or a water-insoluble tertiary amine and its hydrochloride are used, in combination, as a catalyst, thereby ensuring the reaction to proceed at high yield. Moreover, after completion of the reaction, the tertiary amine and, if used, its hydrochloride can be readily collected and reused according to an ordinary procedure in the form of a tertiary amine, thus leading to a reduction in production costs, with an intended product being obtained inexpensively.

## Claims

1. A process for preparing 2-acetylamino-6-chloropurine which comprises reacting in a polar inert solvent 2,9-diacetylguanine or 2-acetylguanine (N²-acetylguanine) with phosphorus oxychloride as a halogenating agent in the presence of a catalyst and subjecting the resulting reaction product to hydrolysis to obtain 2-acetylamino-6-chloropurine, characterized in that the catalyst consists of a combination of a water-insoluble tertiary amine and ammonium chloride or a combination of a water-insoluble tertiary amine and a hydrochloride thereof, wherein the tertiary amine is selected from the group N,N-dimethylaniline, tripropylamine, N,N-diethylaniline or tributylamine.

2. The process according to Claim 1, wherein the molar ratio of the tertiary amine to the 2,9-diacetylguanine or 2-acetylguanine is in the range of 1:1 to 5:1.

3. The process according to Claim 1 or 2, wherein when ammonium chloride is used as one component of the combination, the molar ratio of the ammonium chloride to the 2,9-diacetylguanine or 2-acetylguanine is in the range of 0.5:1 to 5:1.

4. The process according to Claim 1 or 2, wherein when the hydrochloride of the tertiary amine as one component of the combination, the molar ratio of the hydrochloride to the 2,9-diacetylguanine or 2-acetylguanine is in the range of 1:1 to 5:1.

5. The process, according to any of Claims 1 to 4, wherein the molar ratio of the phosphorus oxychloride to the 2,9-diacetylguanine or 2-acetylguanine is in the range of 2:1 to 10:1.

6. The process according to any of Claims 1 to 5, wherein the reaction temperature of the halogenation reaction ranges from 20°C to 80°C.

7. The process according to any of Claims 1 to 6, wherein the polar inert solvent is acetonitrile, tetrahydrofuran or dioxane.

8. The process according to any of Claims 1 to 7, wherein the hydrolysis is effected under conditions of a pH of 2 to 9 and a temperature of 0°C to 70° C.

9. The process according to any of Claims 1 to 8, further comprising subjecting a solution obtained after separation of the reaction product to neutralization and distillation to collect the tertiary amine and, if used, the hydrochloride thereof.

## Patentansprüche

1. Verfahren zur Herstellung von 2-Acetylamino-6-chlorpurin, welches die Umsetzung in einem polaren inerten Lösungsmittel von 2,9-Diacetylguanin oder 2-Acetylguanin (N²-Acetylguanin) mit Phosphoroxychlorid als Halogenierungsmittel in Anwesenheit eines Katalysators und das Unterziehen des resultierenden Reaktionsproduktes der Hydrolyse zum Erhalt von 2-Acetylamino-6-chlorpurin umfaßt, dadurch gekennzeichnet, daß der Katalysator aus einer Kombination eines wasserunlöslichen tertiären Amins und Ammoniumchlorid oder einer Kombination eines wasserunlöslichen tertiären Amins und eines Hydrochlorids hiervon besteht, wobei das tertiäre Amin aus der Gruppe N,N-Dimethylanilin, Tripropylamin, N,N-Diethylanilin oder Tributylamin ausgewählt ist.

2. Verfahren nach Anspruch 1, worin das Molverhältnis des tertiären Amins zu dem 2,9-Diacetylguanin oder 2-Acetylguanin in dem Bereich von 1:1 bis 5:1 liegt.

3. Verfahren nach Anspruch 1 oder 2, worin das Ammoniumchlorid als eine Komponente der Kombination verwendet wird, wobei das Molverhältnis des Ammoniumchlorids zu dem 2,9-Diacetylguanin oder 2-Acetylguanin in dem Bereich von 0,5:1 bis 5:1 liegt.

4. Verfahren nach Anspruch 1 oder 2, worin das Hydrochlorid als eine Komponente der Kombination verwendet wird, wobei das Molverhältnis des Hydrochlorids zu dem 2,9-Diacetylguanin oder 2-Acetylguanin in dem Bereich von 1:1 bis 5:1 liegt.

5. Verfahren nach einem der Ansprüche 1 bis 4, worin das Molverhältnis des Phosphoroxychlorids zu dem 2,9-Diacetylguanin oder 2-Acetylguanin in dem Bereich von 2:1 bis 10:1 liegt.

6. Verfahren nach einem der Ansprüche 1 bis 5, worin die Reaktion der Halogenierungsreaktion von 20°C bis 80°C reicht.

7. Verfahren nach einem der Ansprüche 1 bis 6, worin das polare inerte Lösungsmittel Acetonitril, Tetrahydrofuran oder Dioxan ist.

8. Verfahren nach einem der Ansprüche 1 bis 7, worin die Hydrolyse unter Bedingungen eines pH von 2 bis 9 und einer Temperatur von 0°C bis 70°C durchgeführt wird.

9. Verfahren nach einem der Ansprüche 1 bis 8, weiter umfassend das Unterziehen der nach der Abtrennung des Reaktionsproduktes erhaltenen Lösung der Neutralisation und Destillation zum Sammeln des tertiären Amins und, falls eingesetzt, des Hydrochlorids hiervon.

## Revendications

1. Procédé de préparation de 2-acétylamino-6-chloropurine comprenant les étapes consistant
- à faire réagir dans un solvant polaire inerte de la 2,9-diacétylguanine ou de la 2-acétylguanine (N²-acétylguanine) avec de l'oxychlorure de phosphore, jouant le rôle d'agent d'halogénation, en présence d'un catalyseur, et
- à soumettre le produit réactionnel obtenu à une hydrolyse pour obtenir de la 2-acétylamino-6-chloropurine,
caractérisé par le fait que le catalyseur est constitué d'une combinaison d'une amine tertiaire insoluble dans l'eau et de chlorure d'ammonium, ou d'une combinaison d'une amine tertiaire insoluble dans l'eau et du chlorhydrate de celle-ci, ladite amine tertiaire étant choisie dans le groupe formé par la N,N-diméthylaniline, la tripropylamine, la N,N-diéthylaniline ou la tributylamine.

2. Procédé selon la revendication 1, dans lequel le rapport molaire de l'amine tertiaire à la 2,9-diacétylguanine ou à la 2-acétylguanine est compris entre 1/1 et 5/1.

3. Procédé selon la revendication 1 ou 2, dans lequel on utilise le chlorure d'ammonium comme un des composants de la combinaison, le rapport molaire du chlorure d'ammonium à la 2,9-diacétylguanine ou à la 2-acétylguanine étant compris entre 0,5/1 et 5/1.

4. Procédé selon la revendication 1 ou 2, dans lequel on utilise le chlorhydrate de l'amine tertiaire comme un des composants de la combinaison, le rapport molaire du chlorhydrate à la 2,9-diacétylguanine ou à la 2-acétylguanine étant compris entre 1/1 et 5/1.

5. Procédé selon l'une quelconque des revendications 1 à 4, dans lequel le rapport molaire de l'oxychlorure de phosphore à la 2,9-diacétylguanine ou à la 2-acétylguanine est compris entre 2/1 et 10/1.

6. Procédé selon l'une quelconque des revendications 1 à 5, dans lequel la température réactionnelle de la réaction d'halogénation est comprise entre 20 °C et 80 °C.

7. Procédé selon l'une quelconque des revendications 1 à 6, dans lequel le solvant polaire inerte est l'acétonitrile, le tétrahydrofurane ou le dioxane.

8. Procédé selon l'une quelconque des revendications 1 à 7, dans lequel l'hydrolyse est réalisée à un pH compris entre 2 et 9 et à une température comprise entre 0 et 70 °C.

9. Procédé selon l'une quelconque des revendications 1 à 8, comprenant en outre l'étape consistant à soumettre la solution obtenue après séparation du produit réactionnel à une neutralisation et une distillation de manière à recueillir l'amine tertiaire, et le cas échéant, le chlorhydrate de celle-ci.
